# EUROPEAN PATENT SPECIFICATION

(11) **EP 4 108 662 B1**
(45) Date of publication and mention of the grant of the patent: **10.12.2025**
(21) Application number: 21756547.2
(22) Date of filing: 17.02.2021
(51) Int. Cl.: C07D 413/14, C07D 417/14, H10K 50/844, H10K 59/80, H05B 33/02

(54) **ORGANIC ELECTROLUMINESCENT ELEMENT**
ORGANISCHES ELEKTROLUMINESZENTES ELEMENT
ÉLÉMENT ÉLECTROLUMINESCENT ORGANIQUE

(30) Priority: 17.02.2020 JP 2020023986
(43) Date of publication of application: 28.12.2022
(73) Proprietor: Hodogaya Chemical Co., Ltd., Tokyo, 105-0021 (JP)
(72) Inventor: HIRAYAMA, Yuta, Tokyo 105-0021 (JP); CHIBA, Eriko, Tokyo 105-0021 (JP); KASE, Kouki, Tokyo 105-0021 (JP); YAMAMOTO, Takeshi, Tokyo 105-0021 (JP); SURUGA, Kazuyuki, Tokyo 105-0021 (JP)
(74) Representative: Müller-Boré & Partner Patentanwälte PartG mbB
(86) International application number: PCT/JP2021/005857
(87) International publication number: WO 2021/166935

(56) References cited:
- EP-A1- 4 177 254
- WO-A1-2020/027389
- WO-A1-2021/261851
- WO-A2-2021/085817
- CN-A- 109 761 967
- CN-A- 109 824 659
- KR-B1- 102 059 550

## Description

### TECHNICAL FIELD

The present invention relates to an organic electroluminescent element (hereinafter abbreviated as an organic EL element) which is a self-luminous element suitable for various display devices.

### BACKGROUND

Since an organic EL element is a self-luminous element, the organic EL element is brighter than a liquid crystal element, has excellent visibility, and is able to display clearer images in comparison with a liquid crystal element. For this reason, the organic EL element has been actively studied.

In 1987, C. W. Tang and colleagues at Eastman Kodak developed a laminated structure element using materials assigned different roles. This realized practical applications of an organic EL element with organic materials. These researchers laminated an electron-transporting phosphor and a hole-transporting organic material, and injected both charges into a phosphor layer to cause emission. As a result, a high luminance of 1,000 cd/m² or more at a voltage of 10 V or less (refer to Patent Documents 1 and 2, for example) was obtained.

To date, various improvements have been made for practical applications of the organic EL element. Various roles of the laminated structure are further subdivided to provide an electroluminescence element that includes an anode, a hole injection layer, a hole transport layer, a luminous layer, an electron transport layer, an electron injection layer, and a cathode successively formed on a substrate, wherein high efficiency and durability are achieved by a luminous element of bottom emission structure that emits light from the bottom (refer to Non-Patent Document 1, for example).

In recent years, a luminous element of top emission structure that emits light from the top using metal with a high work function as an anode has been used. The luminous element of bottom emission structure is restricted in terms of luminous area by a pixel circuit. On the other hand, the luminous element of top emission structure has an advantage of having a wide luminous area. In the luminous element of top emission structure, a semitransparent electrode of LiF/Al/Ag (refer to Non-Patent Document 2, for example), Ca/Mg (refer to Non-Patent Document 3, for example), LiF/MgAg, or the like is used as a cathode.

In such a luminous element, when light emitted in a luminous layer enters another film at a given or greater angle, the light is totally reflected at an interface between the luminous layer and the other film. Consequently, only a part of the emitted light is utilized. In recent years, a luminous element provided with a "capping layer" with a high refractive index on the outside of a semitransparent electrode with a low refractive index has been proposed to improve light extraction efficiency (refer to Non-Patent Documents 2 and 3, for example).

The effect of the capping layer in the luminous element of the top emission structure was confirmed using the luminous element using Ir(ppy)₃ as a luminous material. In this luminous element, in the case where there was no capping layer, the current efficiency was 38 cd/A, whereas in the case of the luminous element using ZnSe having a film thickness of 60 nm as the capping layer, the current efficiency was 64 cd/A. It is also indicated that the highest point of transmittance of the semitransparent electrode and the capping layer does not necessarily coincide with the highest point of efficiency. It is indicated that the maximum point of light extraction efficiency is determined by an interference effect (refer to Non-Patent Document 3, for example).

Conventionally, it has been proposed to use a metal mask having high definition for forming a capping layer. However, when the metal mask is used under a high temperature condition, there is a problem in that alignment accuracy decreases because the metal mask is distorted by heat. Therefore, ZnSe cannot be deposited at an accurate position by using a metal mask with high definition, which may affect the luminous element itself, because ZnSe has a high melting point of 1100°C or higher (for example, see Non-Patent Document 3). Furthermore, deposition by sputtering also affects the luminous element. For this reason, a capping layer made of an inorganic material is not suitable for use.

In addition, tris(8-hydroxyquinoline)aluminum (hereinafter referred to as Alq₃) may be used as a capping layer for adjusting the refractive index (for example, see Non-Patent Document 2.). Alq₃ is known as an organic EL material commonly used as a green luminous material or an electron transport material. However, since Alq₃ has a weak absorption near 450 nm, which is used in blue luminous materials, there has been a problem of causing a decrease in color purity and a decrease in light extraction efficiency in the case of the blue luminous element including a capping layer made of Alq₃.

Further, in a conventional element provided with a capping layer, there is a problem in that light having a wavelength of 400 nm to 410 nm of sunlight passes through the element, thereby affecting the material inside the element, and thus decreasing the color purity and the light extraction efficiency. Besides, Patent Document 5 relates to heteroaryl amine structure based organic compounds and applications thereof.

In order to improve the element characteristics of an organic EL element, it is particularly required to absorb light having a wavelength of 400 nm to 410 nm of sunlight so as not to affect the material inside the element. Further, in order to significantly improve the light extraction efficiency, a material having a high absorption coefficient, a high refractive index, and excellent stability and durability of a thin film is required as a material for the capping layer.

[Patent Document 1] Publication of Unexamined Patent Application No. H08-048656
[Patent Document 2] Japanese Patent No. 3194657
[Patent Document 3] WO 2014/009310
[Patent Document 4] WO 2013/038627
[Patent Document 5] CN 109 824 659 A

[Non-Patent Document 1] Proceedings of the 9th Session of the Japan Society of Applied Physics, pages 55 to 61 (2001)
[Non-Patent Document 2] Appl. Phys. Lett., 78, 544 (2001)
[Non-Patent Document 3] Appl. Phys. Lett., 82, 466 (2003)
[Non-Patent Document 4] J. Org. Chem., 71, 1802 (2006)
[Non-Patent Document 5] J. Org. Chem., 60, 7508 (1995))
[Non-Patent Document 6] Synth. Commun., 11, 513 (1981)
[Non-Patent Document 7] Appl. Phys. Lett., 98, 083302 (2011)

### SUMMARY OF THE INVENTION

### [Problems to Be Solved by Invention]

It is an object of the present invention to provide an organic EL element having a capping layer composed of a material having the following characteristics in order to improve the element characteristics of the organic EL element, in particular, to absorb light having a wavelength from 400 nm to 410 nm of sunlight so as not to affect the material inside the element, and to greatly improve the light extraction efficiency.
(1) A high absorption coefficient,
(2) A high refractive index,
(3) Good stability of a thin film,
(4) Excellent durability,
(5) Excellent light resistance, and
(6) No absorption in each of the wavelength regions of blue, green, and red.

A capping layer material suitable for the present invention has the following physical characteristics.
(1) A high absorption coefficient,
(2) A high refractive index,
(3) Vapor deposition capability,
(4) Stable thin film state, and
(5) A high glass transition temperature.

The element suitable for the present invention has the following physical characteristics.
(1) Absorption of light of 400 nm to 410 nm,
(2) High light extraction efficiency,
(3) No degradation in color purity,
(4) Transmission of light without change with time, and
(5) Long life.

### [Means for Solving the Problem]

Therefore, the present inventors have paid attention to the fact that an arylamine-based material is excellent in the stability and durability of the thin film in order to achieve the above object. The amine compound with specific benzoazole ring structure with high refractive index was selected as a material with high absorbance from 400 nm to 410 nm in the absorption spectrum of 10⁻⁵ mol/L concentration. The benzoazole ring structure is a structure obtained by condensing a benzene ring with an azole of a hetero-5-membered ring containing one or more nitrogen atoms. An organic EL element using the compound as a material constituting a capping layer was produced, and the characteristics of the element were intensively evaluated. As a result, the present invention was completed.

That is, according to the present invention, the organic EL elements as defined in the appended set of claims are provided. Subject matter falling outside the scope of the claims is not part of the invention,

In general, the capping layer comprises an amine compound represented by a general formula (1), 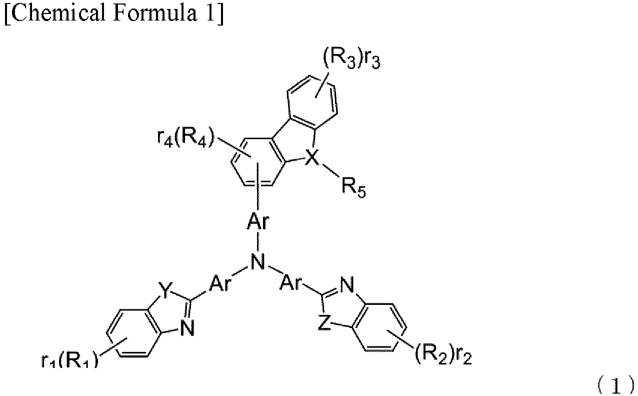 wherein, in the formula, R₁ to R₅ may be the same or different from each other, and each represents a deuterium atom, a fluorine atom, a chlorine atom, a cyano group, a nitro group, a linear or branched alkyl group having 1 to 6 carbon atoms which may have a substituent, a cycloalkyl group having 5 to 10 carbon atoms which may have a substituent, a linear or branched alkenyl group having 2 to 6 carbon atoms which may have a substituent, a linear or branched alkyloxy group having 1 to 6 carbon atoms which may have a substituent, a cycloalkyloxy group having 5 to 10 carbon atoms which may have a substituent, an aromatic hydrocarbon group which may have a substituent, an aromatic heterocyclic group which may have a substituent, a fused polycyclic aromatic group which may have a substituent, or an aryloxy group which may have a substituent;
when there is a plurality of R₁ to R₄, the neighbors may form a ring;
X represents an oxygen atom, a sulfur atom, or a nitrogen atom;
Y and Z represent an oxygen atom or a sulfur atom;
however, when X is an oxygen atom or a sulfur atom, there is no R₅ for X;
Ar may be identical or different from each other, and each represents a divalent group of an aromatic hydrocarbon group which may have a substituent, an aromatic heterocyclic group which may have a substituent, or a fused polycyclic aromatic group which may have a substituent;
r₁ to r₃ are integers of 0 to 4; and
r₄ represents an integer of 0 to 3.

In general, the amine compound is represented by a general formula (1a), 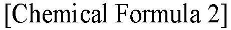 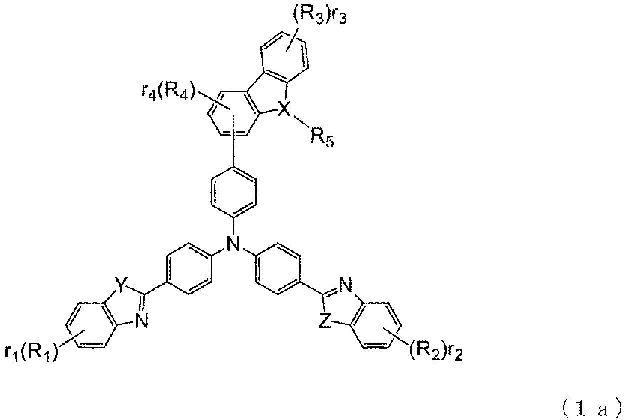 wherein, in the formula, R₁ to R₅, X, Y, Z, and r₁ to r₄ are as defined in the general formula (1).

As the "aromatic hydrocarbon group", the "aromatic heterocyclic group", or the "fused polycyclic aromatic group" in the "aromatic hydrocarbon group which may have a substituent", the "aromatic heterocyclic group which may have a substituent", or the "fused polycyclic aromatic group which may have a substituent" represented by R₁ to R₅, Ar in the general formula (1) or (1a), the "aromatic hydrocarbon group" is an uncondensed ring structure which shows aromaticity and in which atoms constituting the ring have only carbon atoms; the "aromatic heterocyclic group" is a ring structure which shows aromaticity and in which atoms constituting the ring have one or more kinds of atoms other than carbon; and the "fused polycyclic aromatic group" is a ring structure in which a plurality of aromatic rings are condensed and in which atoms constituting the ring have only carbon atoms. Specifically, examples of the "aromatic hydrocarbon group" include a phenyl group, a biphenylyl group, a terphenylyl group, and the like; and also include another aryl group having 6 to 30 carbon atoms, which has an uncondensed ring structure and shows aromaticity. Examples of the "aromatic heterocyclic group" include a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, and the like; and another heteroaryl group having 2 to 20 carbon atoms. Examples of the "fused polycyclic aromatic group" include a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, and the like; and another aryl group having a ring structure having 6 to 30 carbon atoms, in which a plurality of aromatic rings are condensed.

As the "linear or branched alkyl group having 1 to 6 carbon atoms", the "cycloalkyl group having 5 to 10 carbon atoms", the "linear or branched alkenyl group having 2 to 6 carbon atoms", the "linear or branched alkyloxy groups having 1 to 6 carbon atoms", the "cycloalkyloxy group having 5 to 10 carbon atoms", or the "aryloxy group", in the "linear or branched alkyl group having 1 to 6 carbon atoms which may have a substituent", the "cycloalkyl group having 5 to 10 carbon atoms which may have a substituent", the "linear or branched alkenyl group having 2 to 6 carbon atoms which may have a substituent", the "linear or branched alkyloxy groups having 1 to 6 carbon atoms which may have a substituent", the "cycloalkyloxy group having 5 to 10 carbon atoms which may have a substituent", or the "aryloxy group which may have a substituent", represented by R₁ to R₅ in the general formula (1) or (1a), specifically, examples of the "linear or branched alkyl group having 1 to 6 carbon atoms" include a methyl group, an ethyl group, a n-propyl group, an isopropyl group, a n-butyl group, an isobutyl group, a tert-butyl group, a n-pentyl group, an isopentyl group, a neopentyl group, a n-hexyl group, and the like; examples of the "cycloalkyl group having 5 to 10 carbon atoms" include a cyclopentyl group, a cyclohexyl group, a 1-adamantyl group, a 2-adamantyl group, and the like; examples of the "linear or branched alkenyl group having 2 to 6 carbon atoms" include a vinyl group, an allyl group, an isopropenyl group, a 2-butenyl group, and the like; examples of the "linear or branched alkyloxy group having 1 to 6 carbon atoms" include a methyloxy group, an ethyloxy group, and an n-propyloxy group; examples of the "cycloalkyloxy group having 5 to 10 carbon atoms" include a cyclopentyloxy group, a cyclohexyloxy group, a 1-adamantyloxy group, and the like; and examples of the "aryloxy group" include a phenyloxy group, a tolyloxy group, a biphenyloxy group, and the like.

Specifically, examples of "substituent" in the "aromatic hydrocarbon group which may have a substituent", the "aromatic heterocyclic group which may have a substituent", or the "fused polycyclic aromatic group which may have a substituent", the "linear or branched alkyl group having 1 to 6 carbon atoms which may have a substituent", the "cycloalkyl group having 5 to 10 carbon atoms which may have a substituent", the "linear or branched alkenyl group having 2 to 6 carbon atoms which may have a substituent", represented by R₁ to R₅ and Ar in the general formula (1) or (1a), include deuterium atoms, cyano groups, and nitro groups; halogen atoms such as fluorine, chlorine, bromine, and iodine atoms; silyl groups such as trimethylsilyl and triphenylsilyl groups; linear or branched alkyl groups having 1 to 6 carbon atoms, such as methyl, ethyl, or propyl groups; linear or branched alkyloxy groups having 1 to 6 carbon atoms, such as methyloxy, ethyloxy, and propyloxy groups; alkenyl groups such as vinyl and allyl groups; aryloxy groups such as phenyloxy and tolyloxy groups; arylalkyloxy groups such as benzyloxy groups and phenetyloxy groups; aromatic hydrocarbon groups or fused polycyclic aromatic groups such as phenyl, biphenylyl, terphenylyl, naphthyl, anthracenyl, phenanthrenyl, fluorenyl, spirobifluorenyl, indenyl, pyrenyl, perylenyl, fluoranthenyl, and triphenylenyl groups; and pyridyl group, thienyl group, furyl group, pyrrolyl group, quinolyl group, isoquinolyl group, benzofuranyl group, benzothienyl group, indolyl group, carbazolyl group, benzoxazolyl group, benzothiazolyl group, quinoxalinyl group, benzimidazolyl group, pyrazolyl group, dibenzofuranyl group, dibenzothienyl group, and carbolinyl group, as well as other aryl groups having 6 to 30 carbon atoms or other heteroaryl groups having 2 to 20 carbon atoms, and these substituents may further be substituted with the exemplified substituents. Further, these substituents and the substituted benzene ring or the substituents substituted in the same benzene ring may be mutually bonded via a single bond, a methylene group which may have a substituent, an oxygen atom, or a sulfur atom to form a ring.

More specifically, in the organic EL element of the present invention, the capping layer comprises at least one compound selected from the group consisting of a compound (1-1) represented by the following formula (1-1), a compound (1-6) represented by the following formula (1-6), and a compound (1-18) represented by the following formula (1-18)

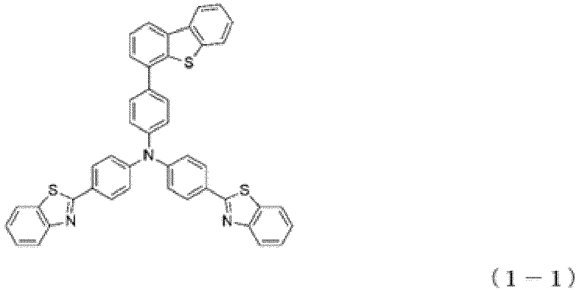

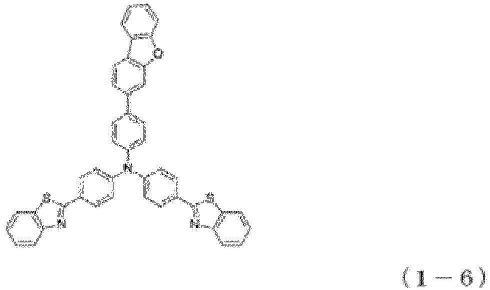

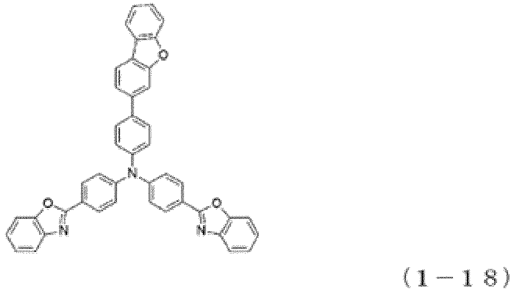

In the organic EL element of the present invention, the thickness of the capping layer is preferably in the range of 30 nm to 120 nm, and more preferably in a range of 40 nm to 80 nm.

**In** the organic EL element of the present invention, the refractive index of the capping layer is preferably 1.90 or more, and more preferably 2.00 or more, within a wavelength range of 500 nm to 570 nm of light passing through the capping layer.

**In** the organic EL element of the present invention, the capping layer may be formed by laminating or mixing two or more different constituent materials.

### [Effect of the Invention]

The organic EL element of the present invention has a capping layer provided outside a transparent or translucent electrode and having a refractive index higher than that of the translucent electrode, so that it is possible to obtain an organic EL element capable of greatly improving light extraction efficiency. Therefore, the light extraction efficiency of each color can be optimized by using the high-definition mask without damaging the luminous element. Further, it can be suitably applied to a full color display, and it is possible to display a clear and bright image with good color purity.

In the organic EL element of the present invention, as a material for the capping layer, a material for the organic EL element having a high absorption coefficient, a high refractive index, an excellent thin film stability, an excellent durability, and an excellent light resistance is used. Therefore, compared with the conventional organic EL element, the color purity is maintained without being affected by sunlight, and the light extraction efficiency can be greatly improved. Furthermore, it is possible to realize an organic EL element having high efficiency and long life.

### BRIEF DESCRIPTION OF DRAWINGS

FIG. 1 is a diagram showing the structure of compounds (1-1) - (1-12) as amine compounds represented by general formulae (1) and (1a).
FIG. 2 is a diagram showing the structure of compounds (1-13) - (1-24) as amine compounds represented by general formulae (1) and (1a).
FIG. 3 is a diagram showing the structure of compounds (1-25) - (1-36) as amine compounds represented by general formulae (1) and (1a).
FIG. 4 is a diagram showing the structure of compounds (1-37) - (1-48) as amine compounds represented by general formulae (1) and (1a).
FIG. 5 is a diagram showing the structure of compounds (1-49) - (1-60) as amine compounds represented by general formulae (1) and (1a).
FIG. 6 is a diagram showing an organic EL element configuration of Examples 12 to 19 and Comparative Examples 1 to 4.

### DETAILED DESCRIPTION OF THE INVENTION

The amine compound represented by the general formula (1) or (1a) is a compound having a benzoazole ring structure. The benzoazole derivative which is the main skeleton of the compound can be synthesized by a known method as described below (for example, see Non-Patent Document 4.). Further, the amine compound represented by the general formula (1) or (1a) can be synthesized by carrying out a coupling reaction of a synthesized halogenated benzoazole derivative and an arylamine by using a copper catalyst, a palladium catalyst, or the like. In addition, by making the halogenated benzoazole derivative into a boronic acid derivative or a boronic acid ester derivative, an amine compound represented by the above general formula (1) or (1a) can be similarly synthesized by a coupling reaction of the boronic acid derivative or the boronic acid ester derivative with an halogenated arylamine (for example, see Non-Patent Documents 5 and 6.).

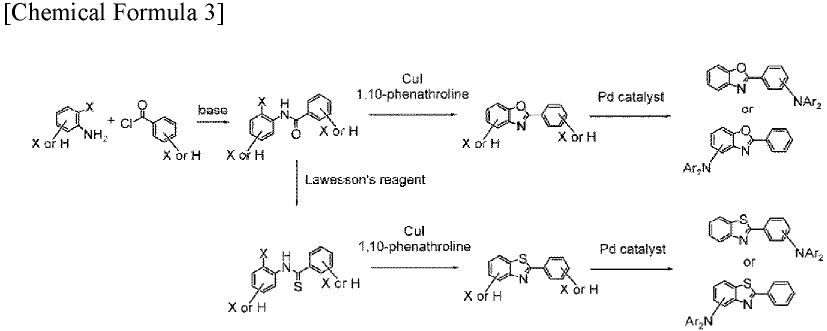

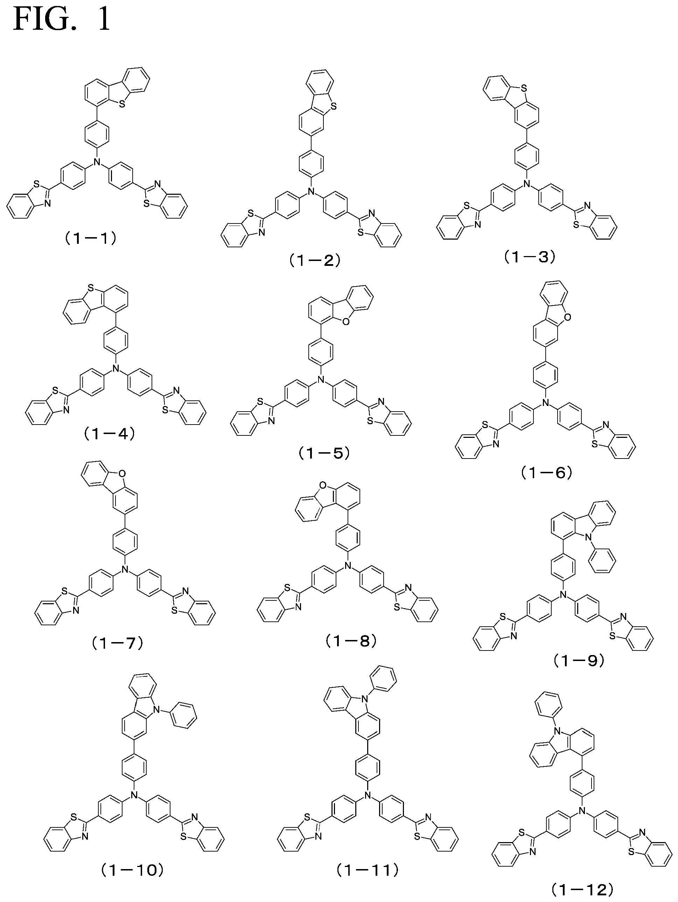

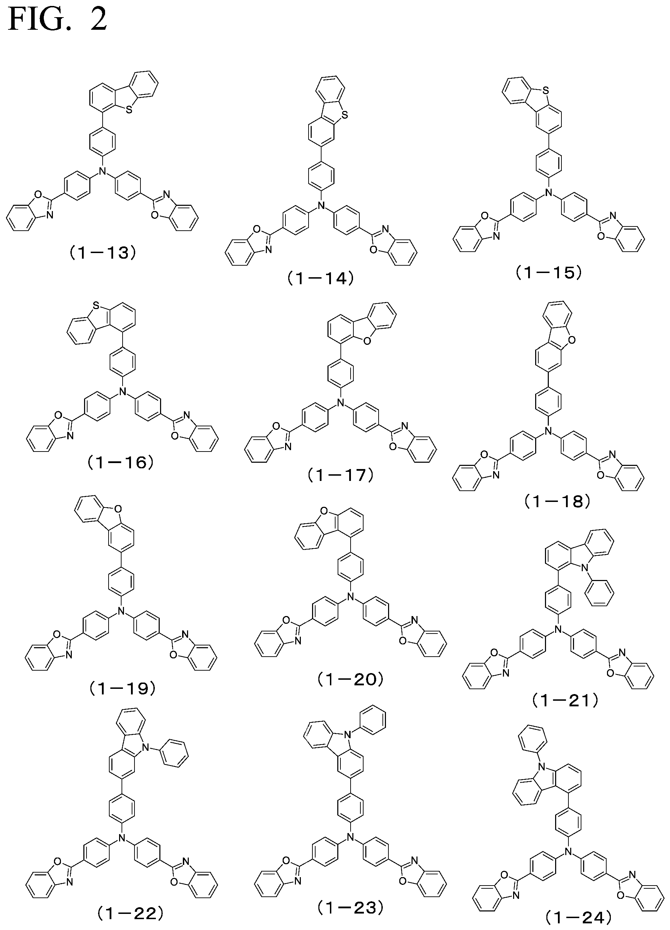

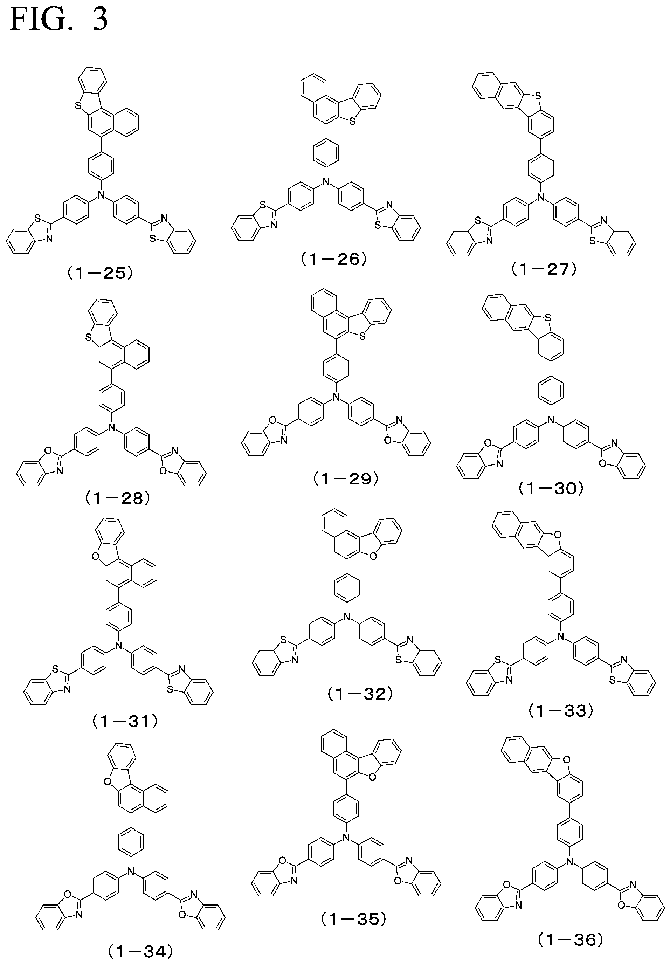

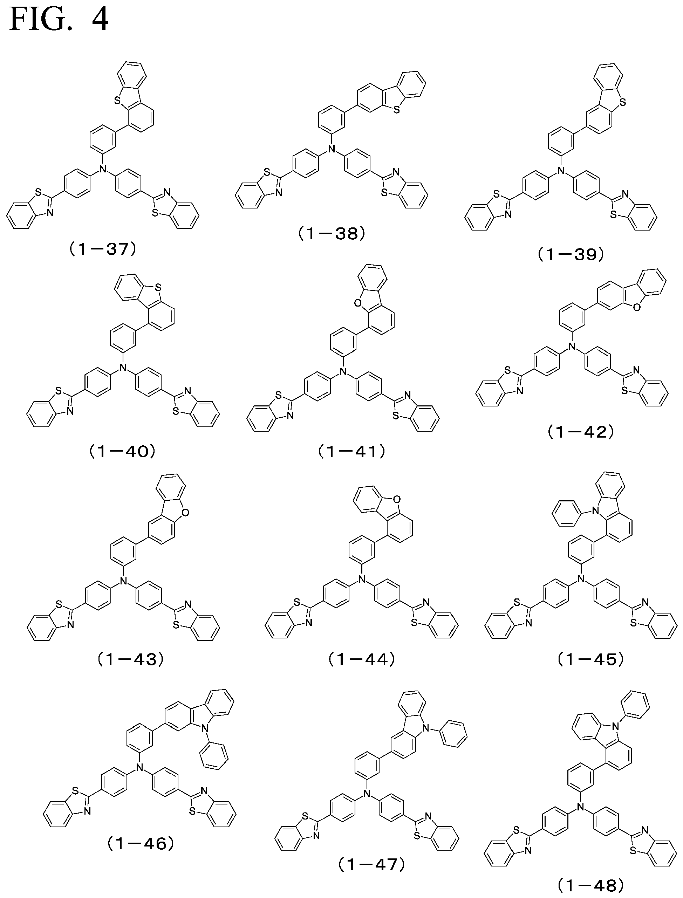

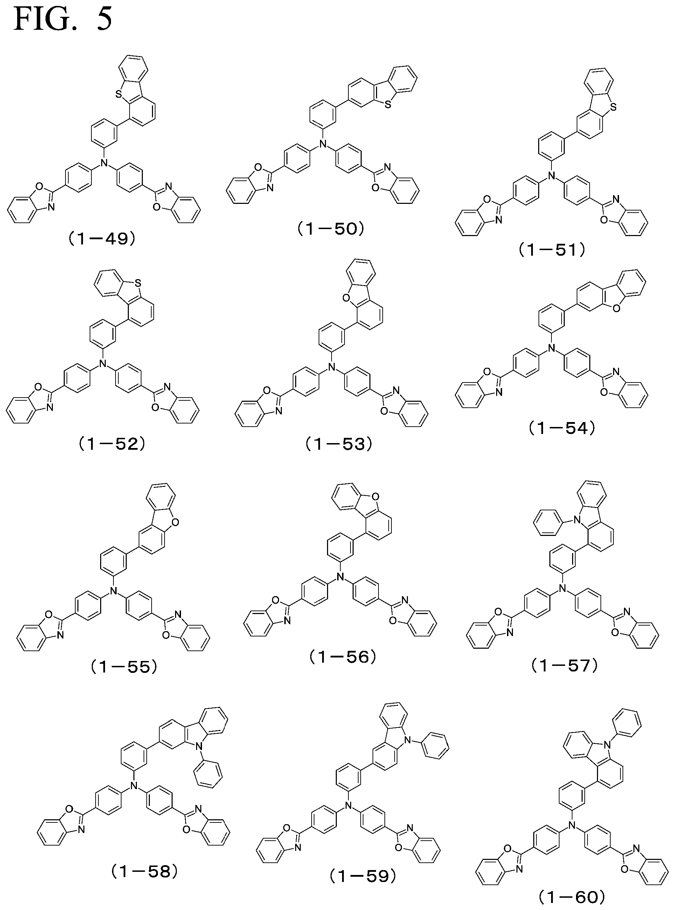

Among the amine compounds represented by the above general formula (1) or (1a), specific examples of compounds are shown in FIGS. 1 to 5.

The compound represented by the general formula (1) or (1a) is purified by a column chromatography purification; an adsorptive purification by using silica gel, activated carbon, activated clay, etc.; recrystallization or crystallization by using a solvent; sublimation purification; or the like. The resulting product was identified by NMR analysis. As a physical characteristic, a melting point, a glass transition point (Tg), and a refractive index were measured. The melting point is an index of vapor deposition property, the glass transition temperature (Tg) is an index of stability of the thin film state, and the refractive index is an index of improvement of light extraction efficiency.

The melting point and the glass transition temperature (Tg) were measured by a sensitive differential scanning calorimeter (manufactured by Bulker AXS, DSC 3100 SA) using a powder.

The refractive index and extinction coefficient were measured using a spectroscopic measuring device (manufactured by Filmetrics, F10-RT-UV) by preparing a thin film having a thickness of 80 nm on a silicon substrate.

The absorbance was measured by adjusting a concentration to 10⁻⁵ mol/L in a toluene solvent. The absorption coefficient was measured using an ultraviolet-visible near-infrared spectrophotometer (manufactured by JASCO, V-650) at 4 different concentrations in toluene solution: 5.0 × 10⁻⁶ mol/L, 1.0 × 10⁻⁵ mol/L, 1.5 × 10⁻⁵ mol/L, and 2.0 × 10⁻⁵ mol/L.

As a structure of the organic EL element, for example, in the case of a luminous element having a top emission structure, a multilayer structure in which an anode, a hole transport layer, a luminous layer, an electron transport layer, a cathode, and a capping layer are sequentially provided on a glass substrate can be used. In addition, a multilayer structure further having a hole injection layer between the anode and the hole transport layer, a multilayer structure further having an electron blocking layer between the hole transport layer and the luminous layer, a multilayer structure further having a hole blocking layer between the luminous layer and the electron transport layer, and a multilayer structure further having an electron injection layer between the electron transport layer and the cathode can be used. In these multilayer structures, it is possible to omit or combine several organic layers. For example, the hole injection layer and the hole transport layer may be combined, the hole transport layer and the electron blocking layer may be combined, the hole blocking layer and the electron transport layer may be combined, or the electron transport layer and the electron injection layer may be combined. It is also possible to have a structure in which two or more organic layers having the same function are laminated. For example, a configuration in which two hole transport layers are stacked, a configuration in which two luminous layers are stacked, a configuration in which two electron transport layers are stacked, a configuration in which two capping layers are stacked, and the like can be used.

A total thickness of each layer of the organic EL element is preferably about 200 nm to 750 nm, and more preferably about 350 nm to 600 nm. The thickness of the capping layer is preferably from 30 nm to 120 nm, and more preferably from 40 nm to 80 nm. In this case, good light extraction efficiency can be obtained. The film thickness of the capping layer can be appropriately changed according to the type of the luminous material used for the luminous element, the thickness of the organic EL element other than the capping layer, and the like.

As the anode of the organic EL element, an electrode material having a large work function such as ITO or gold is used.

As the material of the hole injection layer of the organic EL element, an arylamine compound having three or more triphenylamine structures in a molecule and having a structure in which the three or more triphenylamine structures are linked by a single bond or a divalent group not containing a heteroatom, for example, a material such as a starburst type triphenylamine derivative, and various triphenylamine tetramers; a porphyrin compound represented by copper phthalocyanine; an acceptor type heterocyclic compound such as hexacyanoazatriphenylene; or a coating type polymer material can be used. As the hole injection layer, a single layer formed of these materials alone may be used, or a single layer formed by mixing two or more materials may be used. The hole injection layer may be formed by laminating layers formed of the above materials alone, by laminating layers formed by mixing the materials, or by laminating the layers formed by mixing the materials with the layers formed of the above materials alone. These materials may be formed into a thin film by a vapor deposition method, or may be formed into a thin film by a known method such as a spin coating method or an ink jet method in addition to a vapor deposition method.

As the hole transport layer of the organic EL element, it is preferable to use a benzidine derivative such as N, N'-diphenyl-N, N'-di(m-tolyl)benzidine (hereinafter referred to as TPD), N, N'-diphenyl-N, N'-di (α-naphthyl)benzidine (hereinafter referred to as NPD), N, N, N', N'-tetrabiphenylylbenzidine; 1,1-bis [4-(di-4-tolylamino)phenyl]cyclohexane (hereinafter referred to as TAPC); or the like. According to the present invention, the hole transport layer comprises an arylamine compound having two triphenylamine structures in a molecule and having a structure in which the two triphenylamine structures are linked by a single bond or a divalent group not containing a hetero atom. An example of the arylamine compound includes N, N, N', N'-tetrabiphenylylbenzidine. Further, an arylamine compound having three or more triphenylamine structures in a molecule and having a structure in which the three or more triphenylamine structures are linked by a single bond or a divalent group not containing a hetero atom are preferably used. Examples of the arylamine compound include various triphenylamine trimers and tetramers. A single layer formed of these materials alone or a single layer formed by mixing two or more materials may be used. The hole transport layer may be formed by laminating layers formed of the above materials alone, by laminating layers formed by mixing the materials, or by laminating the layers formed by mixing the materials with the layers formed of the above materials alone. As the material of the hole injection layer, a coating type polymer material such as poly(3,4-ethylenedioxythiophene) (hereinafter referred to as PEDOT)/poly (styrene sulfonate) (hereinafter referred to as PSS) may be used. These materials may be formed into a thin film by a vapor deposition method, or may be formed into a thin film by a known method such as a spin coating method or an ink jet method in addition to a vapor deposition method.

In the hole injection layer, a material which is obtained by further P-doping tris bromophenylamine hexachloroantimony or a radialene derivative in a generally used material of this layer (for example, see Patent Document 3) or a polymer compound having a structure of a benzidine derivative such as TPD in its partial structure may be used.

As the electron blocking layer of the organic EL element, a carbazole derivative such as 4, 4', 4"-tri(N-carbazolyl)triphenylamine (hereinafter referred to as TCTA), 9,9-bis[4-(carbazole-9-yl)phenyl]fluorene, 1,3-bis(carbazole-9-yl)benzene (hereinafter referred to as mCP), 2, 2-bis(4-carbazole-9-ylphenyl)adamantane (hereinafter referred to as Ad-Cz), and a compound having a triarylamine structure and a triphenylsilyl group represented by 9-[4-(carbazole-9-yl)phenyl]-9-[4-(triphenylsilyl)phenyl]-9H-fluorene can be used. These compounds have an electron blocking function. A single layer formed of these materials alone or a single layer formed by mixing two or more materials may be used. The electron blocking layer may be formed by laminating layers formed of the materials alone, by laminating layers formed by mixing the materials, or by laminating layers formed by mixing the materials with the layers formed of these materials alone. These materials may be formed into a thin film by a vapor deposition method, or by a known method such as a spin coating method or an ink jet method in addition to a vapor deposition method.

As the luminous layer of the organic EL element, metal complexes of a quinolinol derivative such as Alq₃, and in addition, various metal complexes, an anthracene derivative, a bisstyrylbenzene derivative, a pyrene derivative, an oxazole derivative, a polyparaphenylenevinylene derivative, or the like can be used. The luminous layer may be composed of a host material and a dopant material. As the host material, an anthracene derivative is preferably used. As the host material, in addition to the luminous material, a heterocyclic compound having an indole ring as a partial structure of a condensed ring, a heterocyclic compound having a carbazole ring as a partial structure of a condensed ring, a carbazole derivative, a thiazole derivative, a benzimidazole derivative, a polydialkylfluorene derivative, or the like can be used. As the dopant material, quinacridone, coumarin, rubrene, perylene and their derivatives, benzopyran derivatives, rhodamine derivatives, aminostyryl derivatives, or the like can be used. And a green luminous material is particularly preferable. A single layer formed of these materials alone or a single layer formed by mixing with other materials may be used. The luminous layer may be formed by laminating layers formed of the materials alone, by laminating layers formed by mixing the materials, or by laminating the layers formed by mixing the materials with the layers formed of these materials alone.

It is also possible to use a phosphorescent emitter as the luminous material. As the phosphorescent emitter, a phosphorescent emitter of a metal complex such as iridium or platinum can be used. For example, green phosphorescent emitters such as Ir(ppy)₃; blue phosphorescent emitters such as FIrpic, FIr6; red phosphorescent emitters such as Btp₂Ir(acac); and the like can be used, and a green phosphorescent light emitter is particularly preferred. As the host material at this time, as a hole injecting host material and electron transporting host material, a carbazole derivative such as 4, 4'-di(N-carbazolyl) biphenyl (hereinafter referred to as CBP), TCTA, or mCP can be used as the host material for hole injection/transport. As the electron transporting host material, p-bis(triphenylsilyl) benzene (hereinafter referred to as UGH2), 2, 2', 2"-(1, 3, 5-phenylene)-tris(1-phenyl-1H-benzimidazole) (hereinafter referred to as TPBI), or the like can be used, and a high-performance organic EL element can be manufactured.

Doping the phosphorescent emitting material into the host material is preferably performed by co-deposition in a range of 1% to 30% by mass with respect to the entire luminous layer to avoid concentration quenching.

As the luminous material, it is also possible to use a material that emits delayed fluorescence, such as PIC-TRZ, CC2TA, PXZ-TRZ, 4CyIPN, CDCB derivatives (for example, see Non-Patent Document 7). These luminous materials may be formed into a thin film by a vapor deposition method, or by a known method such as a spin coating method or an ink jet method in addition to a vapor deposition method.

As the material of the hole blocking layer of the organic EL element, a phenanthroline derivative such as bathocuproine (hereinafter abbreviated as BCP), a metal complex of a quinolinol derivative such as aluminum (III) bis(2-methyl-8-quinolinato)-4-phenylphenolate (hereinafter referred to as BAlq), various rare earth complexes, triazole derivatives, triazine derivatives, pyrimidine derivatives, oxadiazole derivatives, and benzoazole derivatives can be used. The compounds have a hole blocking function. These materials may also serve as materials for the electron transport layer. A single layer formed of these materials alone or a single layer formed by mixing two or more materials may be used. The hole blocking layer may be formed by laminating layers formed of the above materials alone, by laminating layers formed by mixing the materials, or by laminating the layers formed by mixing the materials with the layers formed of these materials alone. These materials may be formed into a thin film by a vapor deposition method, or by a known method such as a spin coating method or an ink jet method in addition to a vapor deposition method.

As the electron transport layer of the organic EL element, in addition to metal complexes of quinolinol derivatives such as Alp₃ and BAlp, various metal complexes, triazole derivatives, triazine derivatives, pyrimidine derivatives, oxadiazole derivatives, pyridine derivatives, benzimidazole derivatives, benzoazole derivatives, thiadiazole derivatives, anthracene derivatives, carbodiimide derivatives, quinoxaline derivatives, pyridoindole derivatives, phenanthroline derivatives, silole derivatives, or the like can be used. A single layer may be formed of these materials, or a single layer may be formed by mixing other materials. The electron transport layer may be formed by laminating layers formed of the materials alone, by laminating layers formed by mixing the materials, or by laminating the layers formed by mixing the materials with the layers formed of these materials alone. These materials may be formed into a thin film by a vapor deposition method, or by a known method such as a spin coating method or an ink jet method in addition to a vapor deposition method.

As the electron injection layer of the organic EL element, an alkali metal salt such as lithium fluoride and cesium fluoride; an alkaline earth metal salt such as magnesium fluoride; a metal complex of a quinolinol derivative such as lithium quinolinol; a metal oxide such as aluminum oxide; a metal such as ytterbium (Yb), samarium (Sm), calcium (Ca), strontium (Sr), cesium (Cs); or the like can be used. The electron injection layer may be omitted in the preferred choice of electron transport layer and cathode.

Further, in the electron injection layer or the electron transport layer, a material in which a metal such as cesium is further N-doped to a material usually used for the layer can be used.

As the cathode of the organic EL element, an electrode material having a low work function such as aluminum; an alloy having a lower work function such as a magnesium-silver alloy, a magnesium-calcium alloy, a magnesium-indium alloy, and an aluminum-magnesium alloy; an electrode material such as ITO and IZO; or the like can be used.

As the capping layer of the organic EL element, it is used an amine compound represented by the general formula (1) or (1a). In particular, the capping layer of the organic EL element comprises at least one compound selected from the group consisting of a compound (1-1) represented by the above formula (1-1), a compound (1-6) represented by the above formula (1-6), and a compound (1-18) represented by the above formula (1-18). A single layer may be formed of these materials, or a single layer may be formed by mixing other materials. The capping layer may be formed by laminating layers formed of the materials alone, by laminating layers formed by mixing the materials, or by laminating the layers formed by mixing the materials with the layers formed of these materials alone. These materials may be formed into a thin film by a vapor deposition method, or by a known method such as a spin coating method or an ink jet method in addition to a vapor deposition method.

Although an organic EL element having a top emission structure has been described above, the present invention is not limited thereto. The present invention can be similarly applied to an organic EL element having a bottom emission structure and an organic EL element having a dual emission structure emitting light from both the top and bottom. In these cases, the electrode in the direction in which the light is extracted from the luminous element is preferably transparent or semitransparent.

The refractive index of the material constituting the capping layer is preferably larger than that of the adjacent electrode. The light extraction efficiency in the organic EL element is improved by the capping layer, and the effect is more effective when a reflectivity at an interface between the capping layer and the material in contact with the capping layer is larger, because the light interference effect is larger when the reflectivity at the interface is larger. Therefore, the refractive index of the material constituting the capping layer is preferably larger than that of the adjacent electrode, and is preferably 1.90 or more, but more preferably 2.00 or more.

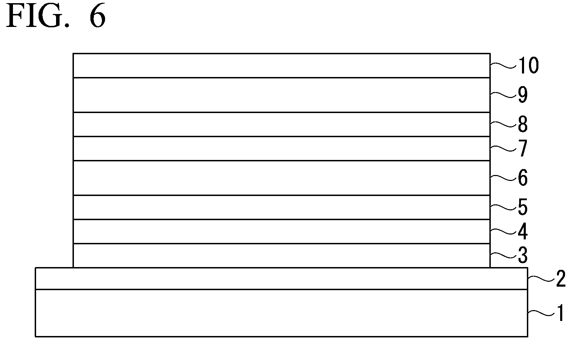

As an example of the method of manufacturing the organic EL element, as shown in FIG. 6, a hole injection layer 3, a first hole transport layer 4, a second hole transport layer 5, a luminous layer 6, an electron transport layer 7, an electron injection layer 8, a cathode 9, and a capping layer 10 are deposited in this order on a glass substrate 1 in which a reflective ITO electrode, as a metal anode 2, is previously formed.

### EXAMPLE

Hereinafter, embodiments of the present invention will be described in detail with reference to examples. The present invention is not limited to the following examples without departing from the scope thereof. In this context, Examples 3 to 7 and 14 to 18 represent reference examples.

### [Example 1]

### <Synthesis of Exemplified Compound (1-18)>

7.0 g of 4-(3-dibenzofuranyl) benzenamine, 16.3 g of 2-(4-bromophenyl) benzoxazole, 7.8 g of t-butoxy sodium, and 70 ml of toluene were added to a reaction vessel, and nitrogen gas was aerated for 30 minutes. 0.7 g of tris(dibenzylideneacetone) dipalladium (0) and 0.7 g of tri-(t-butyl)phosphine in 50% (w/v) toluene were added, and the mixture was stirred overnight under heating reflux.

After the reaction vessel was allowed to cool, dispersion washing was performed at 80°C, the insoluble was filtered, and the filtrate was concentrated to obtain a crude product. A solid precipitated by crystallizing the crude product with a toluene solvent was collected to obtain 12.3 g (yield 70.6%) of yellow powder of the exemplified compound (1-18).

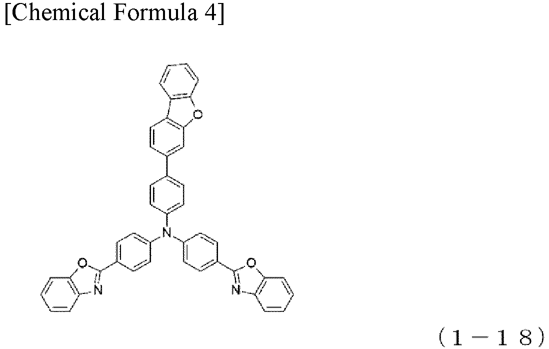

The structure of the obtained yellow powder was identified using NMR.

The following 27 hydrogen signals were detected by ¹H-NMR (CDCl₃). δ (ppm) = 8.23 -8.20 (4 H), 8.05 -7.99 (2 H), 7.84 -7.50 (10 H), 7.41 -7.32 (11 H).

### [Example 2]

### <Synthesis of Exemplified Compound (1-6)>

6.1 g of 4-(3-dibenzofuranyl) benzenamine, 15.0 g of 2-(4-bromophenyl)benzothiazole, 6.8 g of t-butoxy sodium, and 60 ml of toluene were added to a reaction vessel, and nitrogen gas was aerated for 30 minutes. 0.7 g of tris(dibenzylideneacetone) dipalladium (0) and 0.6 g of tri-(t-butyl) phosphine in 50% (w/v) toluene solution were added, and the mixture was stirred under heating reflux overnight.

After the reaction vessel was allowed to cool, dispersion washing was performed at 80°C, the insoluble was filtered, and the filtrate was concentrated to obtain a crude product. A solid precipitated by crystallizing the crude product with a toluene solvent was collected to obtain 9.1 g (yield 57.1%) of yellow powder of the exemplified compound (1-6).

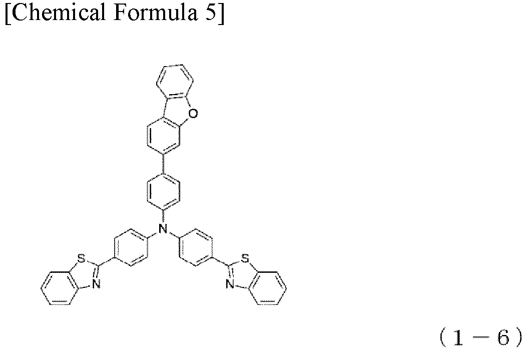

The structure of the obtained yellow powder was identified using NMR.

The following 27 hydrogen signals were detected by ¹H-NMR (CDCl₃).

δ (ppm) = 8.09 -8.20 (8 H), 7.94 -7.91 (2 H), 7.83 (1 H), 7.72 -7.61 (4 H), 7.52 -7.49 (3 H), 7.42 -7.30 (9 H).

### [Example 3]

### <Synthesis of Exemplified Compound (1-17)>

5.3 g of 4-(4-dibenzofuranyl)benzenamine, 12.3 g of 2-(4-bromophenyl)benzoxazole, 5.9 g of t-butoxy sodium, and 50 ml of toluene were added to a reaction vessel, and nitrogen gas was aerated for 30 minutes. 0.6 g of tris(dibenzylideneacetone)dipalladium (0) and 0.5 g of tri-(t-butyl) phosphine in 50% (w/v) toluene solution were added, and the mixture was stirred under heating reflux overnight.

After the reaction vessel was allowed to cool, dispersion washing was performed at 80°C, the insoluble was filtered, and the filtrate was concentrated to obtain a crude product. A solid precipitated by crystallizing the crude product with a toluene solvent was collected to obtain 6.8 g (yield 51.5%) of yellow powder of the exemplified compound (1-17).

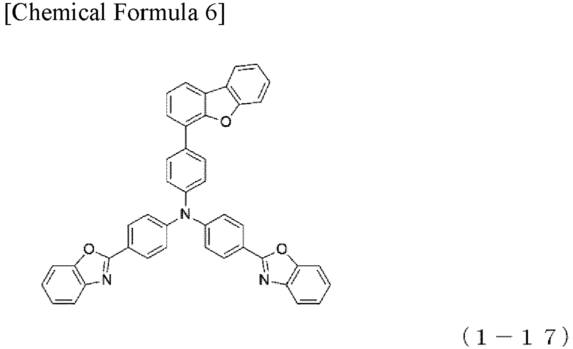

The structure of the obtained yellow powder was identified using NMR.

The following 27 hydrogen signals were detected by ¹H-NMR (CDCl₃).

δ (ppm) = 8.24 -8.20 (4 H), 8.05 -7.96 (4 H), 7.80 -7.77 (2 H), 7.68 -7.59 (4 H), 7.51 -7.36 (13 H).

### [Example 4]

### <Synthesis of Exemplified Compound (1-5)>

5.3 g of 4-(4-dibenzofuranyl)benzenamine, 13.1 g of 2-(4-bromophenyl)benzothiazole, 5.9 g of t-butoxy sodium, and 100 ml of toluene were added to a reaction vessel, and nitrogen gas was aerated for 30 minutes. 0.6 g of tris(dibenzylideneacetone)dipalladium (0) and 0.5 g of tri-(t-butyl) phosphine in 50% (w/v) toluene solution were added, and the mixture was stirred under heating reflux overnight.

After the reaction vessel was allowed to cool, dispersion washing was performed at 80°C, the insoluble was filtered, and the filtrate was concentrated to obtain a crude product. A solid precipitated by crystallizing the crude product with a toluene solvent was collected to obtain 7.5 g (yield 54.15%) of yellow powder of the exemplified compound (1-5).

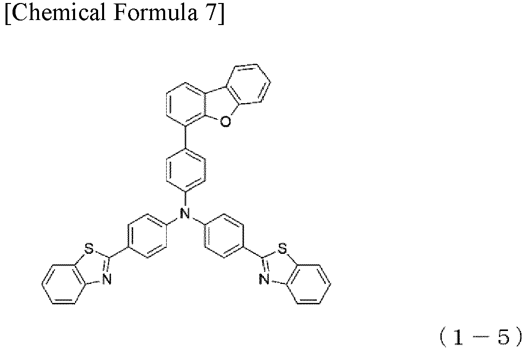

The structure of the obtained yellow powder was identified using NMR.

The following 27 hydrogen signals were detected by ¹H-NMR (CDCl₃).

δ (ppm) = 8.10 -7.91 (12 H), 7.68 -7.64 (2 H), 7.54 -7.33 (13 H).

### [Example 5]

### <Synthesis of Exemplified Compound (1-19)>

7.0 g of 4-(2-dibenzofuranyl)benzenamine, 16.3 g of 2-(4-bromophenyl) benzoxazole, 7.8 g of t-butoxy sodium, and 70 ml of toluene were added to a reaction vessel, and nitrogen gas was aerated for 30 minutes. 0.7 g of tris(dibenzylideneacetone)dipalladium (0) and 0.7 g of tri-(t-butyl) phosphine in 50% (w/v) toluene solution were added, and the mixture was stirred under heating reflux overnight.

After the reaction vessel was allowed to cool, dispersion washing was performed at 80°C, the insoluble was filtered, and the filtrate was concentrated to obtain a crude product. A solid precipitated by crystallizing the crude product with a toluene solvent was collected to obtain 9.9 g (yield 56.9%) of yellow powder of the exemplified compound (1-19).

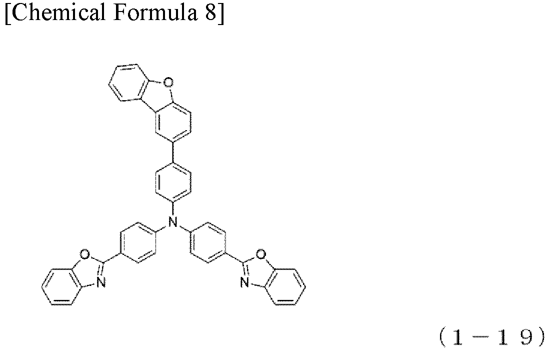

The structure of the obtained yellow powder was identified using NMR.

The following 27 hydrogen signals were detected by ¹H-NMR (CDCl₃).

δ (ppm) = 8.24 -8.19 (5 H), 8.05 -8.02 (1 H), 7.80 -7.58 (9 H), 7.54 -7.49 (1 H), 7.43 -7.32 (11 H).

### [Example 6]

### <Synthesis of Exemplified Compound (1-7)>

5.0 g of 4-(2-dibenzofuranyl)benzenamine, 12.3 g of 2-(4-bromophenyl)benzothiazole, 5.6 g of t-butoxy sodium, and 50 ml of toluene were added to a reaction vessel, and nitrogen gas was aerated for 30 minutes. 0.5 g of tris(dibenzylideneacetone)dipalladium (0) and 0.5 g of tri-(t-butyl) phosphine in 50% (w/v) toluene solution were added, and the mixture was stirred under heating reflux overnight.

After the reaction vessel was allowed to cool, dispersion washing was performed at 80°C, the insoluble was filtered, and the filtrate was concentrated to obtain a crude product. A solid precipitated by crystallizing the crude product with a monochlorobenzene solvent was collected to obtain 8.5 g (yield 65.0%) of yellow powder of the exemplified compound (1-7).

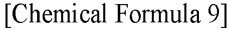

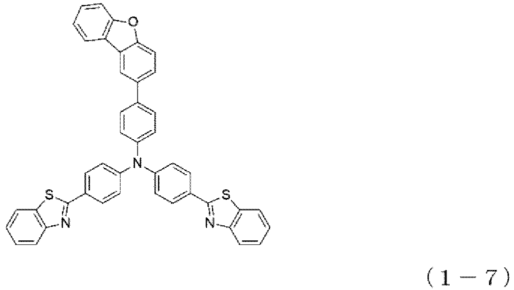

The structure of the obtained yellow powder was identified using NMR.

The following 27 hydrogen signals were detected by ¹H-NMR (CDCl₃).

δ (ppm) = 8.20 -8.19 (1 H), 8.09 -8.03 (6 H), 7.94 -7.91 (2 H), 7.75 -7.61 (5 H), 7.54 -7.49 (3 H), 7.43 -7.30 (10 H).

### [Example 7]

### <Synthesis of Exemplified Compound (1-13)>

5.0 g of 4-(4-dibenzothienyl) benzenamine, 11.0 g of 2-(4-bromophenyl) benzoxazole, 5.2 g of t-butoxy sodium, and 50 ml of toluene were added to a reaction vessel, and nitrogen gas was aerated for 30 minutes. 0.5 g of tris(dibenzylideneacetone)dipalladium (0) and 0.4 g of tri-(t-butyl) phosphine in 50% (w/v) toluene solution were added, and the mixture was stirred under heating reflux overnight.

After the reaction vessel was allowed to cool, dispersion washing was performed at 80°C, the insoluble was filtered, and the filtrate was concentrated to obtain a crude product. A solid precipitated by crystallizing the crude product with a toluene solvent was collected to obtain 10.6 g (yield 88.2%) of yellow powder of the exemplified compound (1-13).

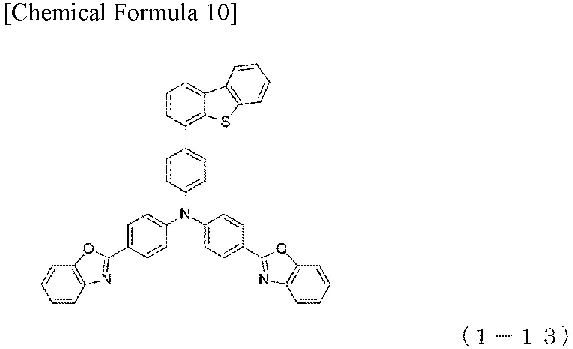

The structure of the obtained yellow powder was identified using NMR.

The following 27 hydrogen signals were detected by ¹H-NMR (CDCl₃).

δ (ppm) = 8.25 -8.18 (6 H), 7.91 -7.88 (1 H), 7.81 -7.78 (4 H), 7.63 -7.49 (6 H), 7.41 -7.34 (10 H).

### [Example 8]

### <Synthesis of Exemplified Compound (1-1)>

5.0 g of 4-(4-dibenzothienyl) benzenamine, 11.6 g of 2-(4-bromophenyl)benzothiazole, 5.2 g of t-butoxy sodium, and 50 ml of toluene were added to a reaction vessel, and nitrogen gas was aerated for 30 minutes. 0.5 g of tris(dibenzylideneacetone)dipalladium (0) and 0.4 g of tri-(t-butyl) phosphine in 50% (w/v) toluene solution were added, and the mixture was stirred under heating reflux overnight.

After the reaction vessel was allowed to cool, dispersion washing was performed at 80°C, the insoluble was filtered, and the filtrate was concentrated to obtain a crude product. A solid precipitated by crystallizing the crude product with a monochlorobenzene solvent was collected to obtain 9.1 g (yield 72.22%) of yellow powder of the exemplified compound (1-1).

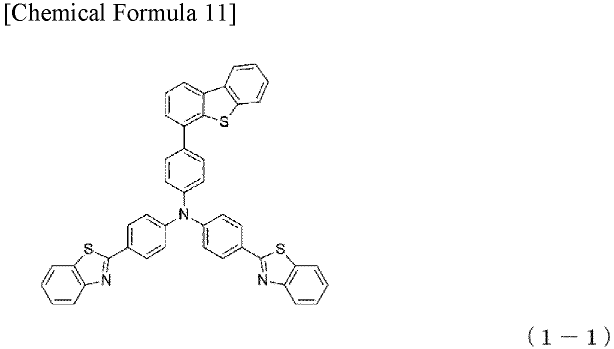

The structure of the obtained yellow powder was identified using NMR.

The following 27 hydrogen signals were detected by ¹H-NMR (CDCl₃).

δ (ppm) = 8.24 -8.18 (2 H), 8.10 -8.06 (6 H), 7.94 -7.88 (3 H), 7.79 -7.76 (2 H), 7.63 -7.49 (6 H), 7.43 -7.33 (8 H).

### [Example 9]

A melting point and a glass transition point of an amine compound represented by general formula (1) or (1a) were measured by a highly sensitive differential scanning calorimeter (manufactured by Bulker AXS, DSC 3100 SA).

| | melting point | glass transition point |
|---|---|---|
| Compound of Example 1 | - °C | 121°C |
| Compound of Example 2 | - °C | 119°C |
| Compound of Example 3 | - °C | 125°C |
| Compound of Example 4 | - °C | 122°C |
| Compound of Example 5 | - °C | 121°C |
| Compound of Example 6 | - °C | 118°C |
| Compound of Example 7 | 247°C | 127°C |
| Compound of Example 8 | - °C | 126°C |

The amine compound represented by the general formula (1) or (1a) has a glass transition temperature of 100°C or higher, indicating that the thin film state is stable.

### [Example 10]

A vapor deposited film having a thickness of 80 nm was prepared on a silicon substrate by using an amine compound represented by the general formula (1) or (1a). Regarding these prepared samples, a refractive index n at each wavelength of 400 nm, 410 nm, 500 nm, and 570 nm and an extinction coefficient k at each wavelength of 400 nm and 410 nm were measured by using a spectrometer (Filmetrics, F10-RT-UV). For comparison, comparative compounds (2-1), (2-2), (2-3), and (2-4) represented by the following structural formulae were also measured (for example, see Patent Document 4). The measurement results are summarized in Table 1.

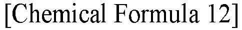

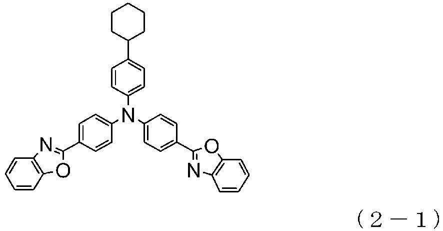

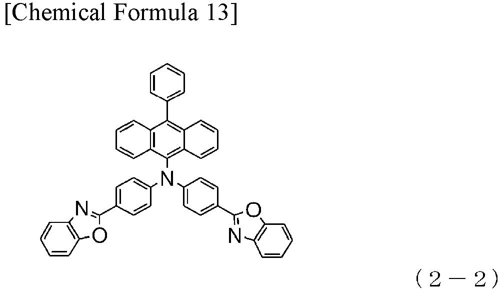

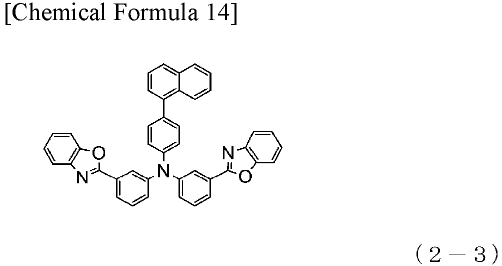

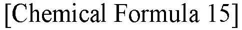

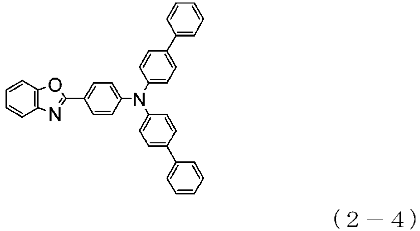

**[Table 1]**

| | Refractive index n (λ: 400 nm) | Refractive index n (λ: 410 nm) | Refractive index n (λ: 500 nm) | Refractive index n (λ: 570 nm) | Extinction coefficient k (λ: 400 nm) | Extinction coefficient k (λ: 410 nm) |
|---|---|---|---|---|---|---|
| Exemplified Compound (1-18) | 2.65 | 2.80 | 2.14 | 2.02 | 0.99 | 0.66 |
| Exemplified Compound (1-6) | 2.29 | 2.56 | 2.19 | 2.06 | 1.08 | 0.94 |
| Exemplified Compound (1-17) | 2.52 | 2.67 | 2.07 | 1.97 | 0.92 | 0.63 |
| Exemplified Compound (1-5) | 2.21 | 2.41 | 2.14 | 2.01 | 0.94 | 0.82 |
| Exemplified Compound (1-19) | 2.57 | 2.72 | 2.10 | 1.99 | 0.90 | 0.62 |
| Exemplified Compound (1-7) | 2.23 | 2.39 | 2.15 | 2.02 | 0.84 | 0.73 |
| Exemplified Compound (1-13) | 2.52 | 2.65 | 2.08 | 1.99 | 0.80 | 0.53 |
| Exemplified Compound (1-1) | 2.30 | 2.51 | 2.16 | 2.05 | 0.88 | 0.75 |
| Comparative Compound (2-1) | 2.05 | 2.04 | 1.88 | 1.84 | 0.05 | 0.03 |
| Comparative Compound (2-2) | 2.35 | 2.27 | 1.94 | 1.88 | 0.14 | 0.06 |
| Comparative Compound (2-3) | 2.30 | 2.20 | 1.91 | 1.87 | 0.45 | 0.31 |
| Comparative Compound (2-4) | 2.37 | 2.31 | 1.87 | 1.80 | 0.32 | 0.11 |

As described above, the compounds (1-1), (1-5), (1-6), (1-7), (1-13), (1-17), (1-18), and (1-19) have a refractive index of 1.90 or higher at a wavelength of 500 nm to 570 nm, and have a value equal to or higher than that of the comparative compounds (2-1), (2-2), (2-3), and (2-4), and this is expected to improve the light extraction efficiency in the organic EL element. In addition, the comparative compounds (2-1), (2-2), (2-3), and (2-4) had extinction coefficients from 0.03 to 0.45 at wavelengths from 400 nm to 410 nm, whereas the compounds (1-1), (1-5), (1-6), (1-7), (1-13), (1-17), (1-18), and (1-19) had extinction coefficients from 0.53 to 1.08. This indicates that the compounds (1-1), (1-5), (1-6), (1-7), (1-13), (1-17), (1-18), and (1-19) absorb the light at wavelengths from 400 nm to 410 nm of sunlight well and the light does not affect the material inside the element.

### [Example 11]

An absorbance of the compounds (1-1), (1-5), (1-6), (1-7), (1-13), (1-17), (1-18), and (1-19) was measured at wavelengths of 400 nm and 410 nm by adjusting a concentration of the compound to 10⁻⁵ mol/L in toluene solution. After adjusting concentrations of the compound to the 4 different concentrations of 5 × 10⁻⁶ mol/L, 1 × 10⁻⁵ mol/L, 1.5 × 10⁻⁵ mol/L, and 2.0 × 10⁻⁵ mol/L in toluene solution, an absorbance coefficient was calculated from the calibration curve obtained by measuring the 4 different concentrations of the compound in toluene solution using an ultraviolet-visible near-infrared spectrometer (made by JASCO, V-650). For comparison, the comparative compounds (2-1), (2-2), (2-3), and (2-4) represented by the above structural formulae were also measured. The measurement results are summarized in Table 2.

**[Table 2]**

| | Peak wavelength (λmax) | Absorbance (λ: 400 nm) | Absorbance (λ: 410 nm) | Absorption coefficient |
|---|---|---|---|---|
| Exemplified Compound (1-18) | 372 nm | 0.76 | 0.28 | 123234 |
| Exemplified Compound (1-6) | 378 nm | 1.21 | 0.95 | 138753 |
| Exemplified Compound (1-17) | 373 nm | 0.79 | 0.27 | 120059 |
| Exemplified Compound (1-5) | 375 nm | 1.23 | 1.00 | 130062 |
| Exemplified Compound (1-19) | 384 nm | 1.02 | 0.38 | 133851 |
| Exemplified Compound (1-7) | 395 nm | 1.38 | 1.18 | 139636 |
| Exemplified Compound (1-13) | 374 nm | 0.74 | 0.58 | 105333 |
| Exemplified Compound (1-1) | 389 nm | 1.15 | 0.90 | 119428 |
| Comparative Compound (2-1) | 305 nm | 0.02 | 0.01 | 114814 |
| Comparative Compound (2-2) | 305 nm | 0.02 | 0.00 | 98970 |
| Comparative Compound (2-3) | 345 nm | 0.60 | 0.21 | 100841 |
| Comparative Compound (2-4) | 373 nm | 0.28 | 0.08 | 88182 |

Absorbance of the comparative compounds (2-1), (2-2), (2-3), and (2-4) at a wavelength of 400 nm is between 0.02 and 0.60, whereas that of the compounds (1-1), (1-5), (1-6), (1-7), (1-13), (1-17), (1-18), and (1-19) is between 0.74 and 1.38. The compounds (1-1), (1-5), (1-6), (1-7), (1-13), (1-17), (1-18), and (1-19) have larger values. The absorbance of the comparative compounds (2-1), (2-2), (2-3), and (2-4) at a wavelength of 410 nm is between 0.00 and 0.21, whereas that of the compounds (1-1), (1-5), (1-6), (1-7), (1-13), (1-17), (1-18), and (1-19) is between 0.27 and 1.18. The compounds (1-1), (1-5), (1-6), (1-7), (1-13), (1-17), (1-18), and (1-19) have larger values. This indicates that the compounds (1-1), (1-5), (1-6), (1-7), (1-13), (1-17), (1-18), and (1-19) can absorb light having a wavelength of 400 nm to 410 nm of sunlight well, and that an absorption coefficient of the compounds (1-1), (1-5), (1-6), (1-7), (1-13), (1-17), (1-18), and (1-19) has a value of 100,000 or more. That is, the compounds (1-1), (1-5), (1-6), (1-7), (1-13), (1-17), (1-18), and (1-19) absorb light well under the same concentration conditions. The compounds (1-1), (1-5), (1-6), (1-7), (1-13), (1-17), (1-18), and (1-19) also absorb light with increasing thickness of the thin film, indicating that the material is excellent in light resistance.

### [Example 12]

As shown in FIG. 6, the organic EL element was prepared by depositing a hole injection layer 3, a first hole transport layer 4, a second hole transport layer 5, a luminous layer 6, an electron transport layer 7, an electron injection layer 8, a cathode 9, and a capping layer 10, in this order, on a glass substrate 1 on which a reflective ITO electrode, as a metal anode 2, was previously formed.

Specifically, the glass substrate 1, on which ITO having a film thickness of 50 nm, a reflective film of silver alloy having a film thickness of 100 nm, and ITO having a film thickness of 5 nm were sequentially formed, was subjected to ultrasonic cleaning in isopropyl alcohol for 20 minutes, and then was dried for 10 minutes on a hot plate heated to 250°C. After UV ozone treatment was performed for 2 minutes, the glass substrate with ITO was attached to a vacuum vapor deposition apparatus, and then, the pressure of the vacuum vapor deposition apparatus was reduced to 0.001 Pa or less. Subsequently, as the hole injection layer 3, an electron acceptor (Acceptor-1) represented by the following structural formula and a compound (3-1) represented by the following structural formula were subjected to binary deposition at a deposition rate at which the deposition rate ratio was Acceptor-1: compound (3-1) = 3: 97 so as to cover the metal anode 2, thereby forming a film thickness of 10 nm. On the hole injection layer 3, the compound (3-1) represented by the following structural formula was subjected to deposition to form the first hole transport layer 4 having a film thickness of 70 nm. On the first hole transport layer 4, a compound (3-2) represented by the following structural formula was subjected to deposition to form the second hole transport layer 5 having a film thickness of 10 nm. On the second hole transport layer 5, a compound (3-3) represented by the following structural formula and a compound (3-4) represented by the following structural formula were subjected to binary deposition at a deposition rate at which the deposition rate ratio was (3-3): (3-4) = 5: 95 to form the luminous layer 6 having a film thickness of 40 nm. On the luminous layer 6, a compound (3-5) represented by the following structural formula and a compound (3-6) represented by the following structural formula were subjected to binary deposition at a deposition rate at which the deposition rate ratio was (3-5): (3-6) = 50: 50 to form the electron transport layer 7 having a film thickness of 30 nm. On the electron transport layer 7, lithium fluoride was subjected to deposition to form the electron injection layer 8 having a film thickness of 1 nm.

On the electron injection layer 8, a magnesium silver alloy was subjected to deposition to form the cathode 9 having a film thickness of 12 nm. Finally, the compound (1-18) of Example 1 was subjected to deposition to form the capping layer 10 having a film thickness of 60 nm. The characteristics of the fabricated organic EL element were measured in the atmosphere at room temperature.

The measurement results of emission characteristics by applying DC voltage to the fabricated organic EL element are summarized in Table 3.

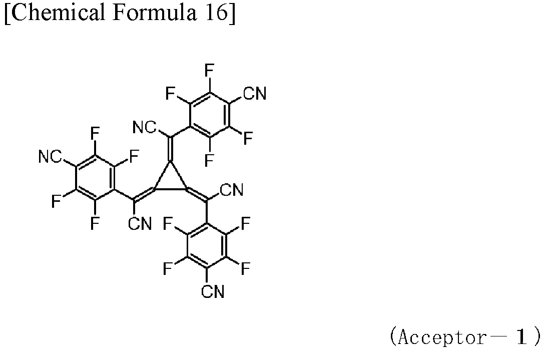

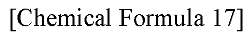

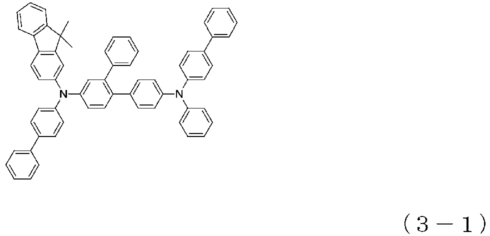

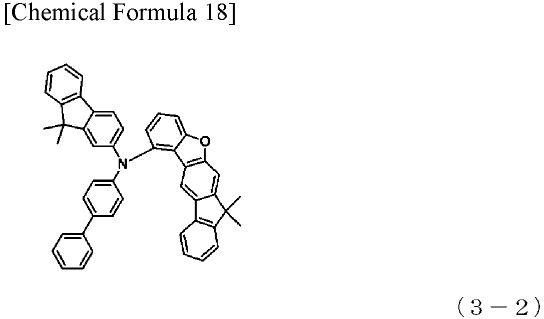

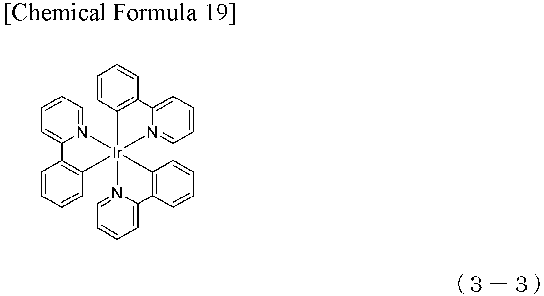

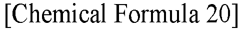

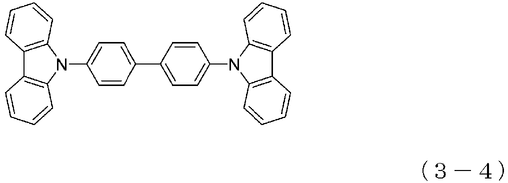

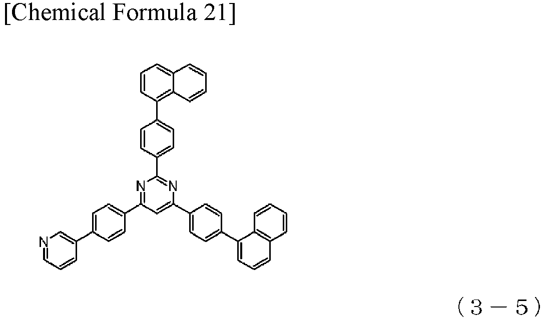

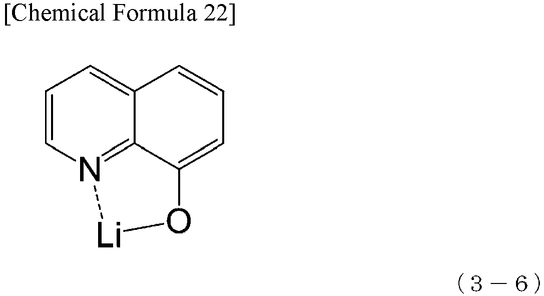

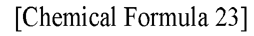

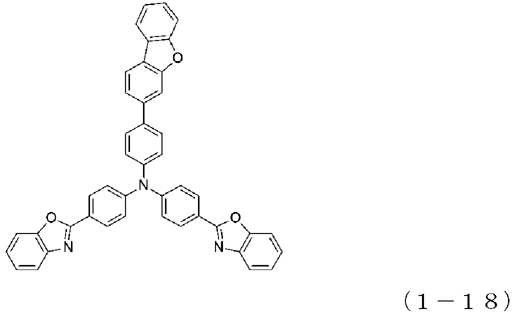

### [Example 13]

An organic EL element was produced under the same conditions as in Example 12, except that the compound (1-6) of Example 2 was used instead of the compound (1-18) of Example 1 as the material of the capping layer 10. The characteristics of the fabricated organic EL element were measured in the atmosphere at room temperature. The measurement results of emission characteristics when a DC voltage was applied to the fabricated organic EL element are summarized in Table 3.

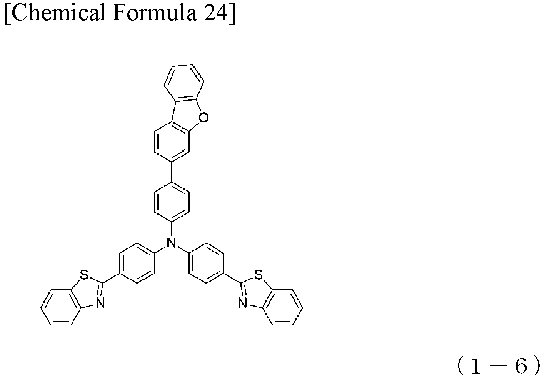

### [Example 14]

An organic EL element was produced under the same conditions as in Example 12, except that the compound (1-17) of Example 3 was used instead of the compound (1-18) of Example 1 as the material of the capping layer 10. The characteristics of the fabricated organic EL element were measured in the atmosphere at room temperature. The measurement results of emission characteristics when a DC voltage was applied to the fabricated organic EL element are summarized in Table 3.

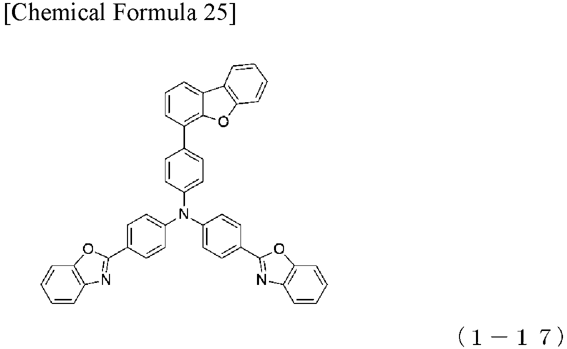

### [Example 15]

An organic EL element was produced under the same conditions as in Example 12, except that the compound (1-5) of Example 4 was used instead of the compound (1-18) of Example 1 as the material of the capping layer 10. The characteristics of the fabricated organic EL element were measured in the atmosphere at room temperature. The measurement results of emission characteristics when a DC voltage was applied to the fabricated organic EL element are summarized in Table 3.

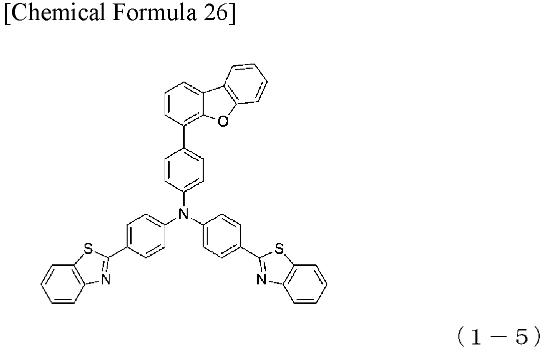

### [Example 16]

An organic EL element was produced under the same conditions as in Example 12, except that the compound (1-19) of Example 5 was used instead of the compound (1-18) of Example 1 as the material of the capping layer 10. The characteristics of the fabricated organic EL element were measured in the atmosphere at room temperature. The measurement results of emission characteristics when a DC voltage was applied to the fabricated organic EL element are summarized in Table 3.

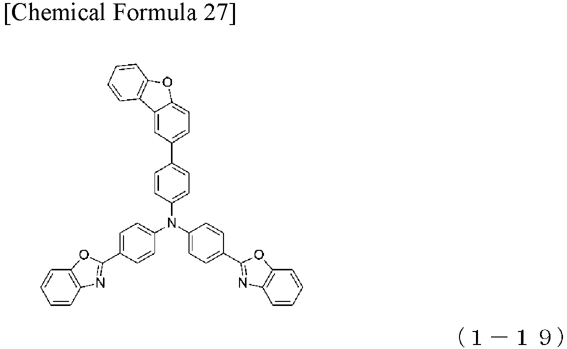

### [Example 17]

An organic EL element was produced under the same conditions as in Example 12, except that the compound (1-7) of Example 6 was used instead of the compound (1-18) of Example 1 as the material of the capping layer 10. The characteristics of the fabricated organic EL element were measured in the atmosphere at room temperature. The measurement results of emission characteristics when a DC voltage was applied to the fabricated organic EL element are summarized in Table 3.

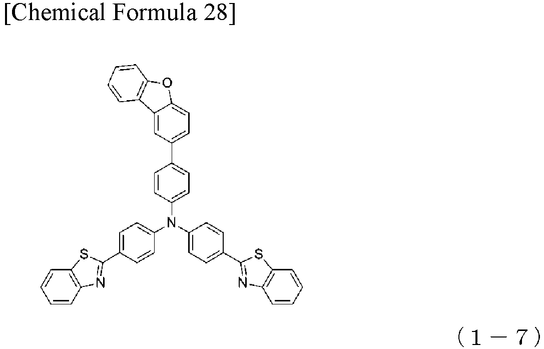

### [Example 18]

An organic EL element was produced under the same conditions as in Example 12, except that the compound (1-13) of Example 7 was used instead of the compound (1-18) of Example 1 as the material of the capping layer 10. The characteristics of the fabricated organic EL element were measured in the atmosphere at room temperature. The measurement results of emission characteristics when a DC voltage was applied to the fabricated organic EL element are summarized in Table 3.

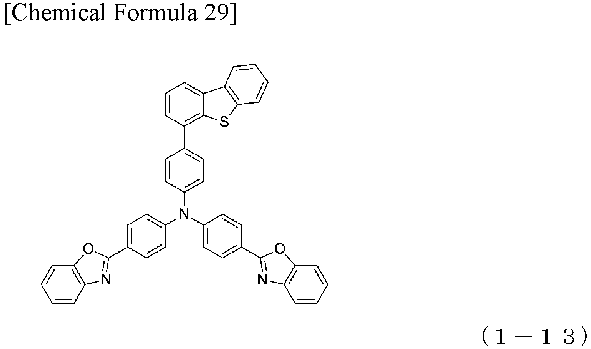

### [Example 19]

An organic EL element was produced under the same conditions as in Example 12, except that the compound (1-1) of Example 8 was used instead of the compound (1-18) of Example 1 as the material of the capping layer 10. The characteristics of the fabricated organic EL element were measured in the atmosphere at room temperature. The measurement results of emission characteristics when a DC voltage was applied to the fabricated organic EL element are summarized in Table 3.

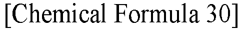

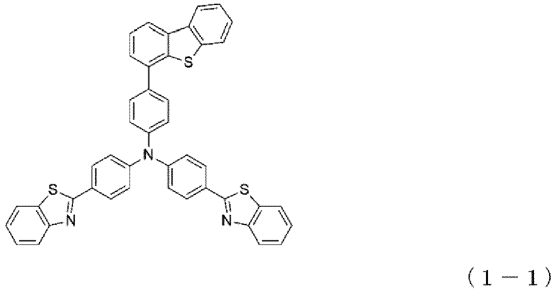

### [Comparative Example 1]

For comparison, an organic EL element was prepared under the same conditions as in Example 7, except that a comparative compound (2-1) represented by the following structural formula was used instead of the compound (1-18) of Example 1 to form the capping layer 10 having a film thickness of 60 nm. The characteristics of the fabricated organic EL element were measured in the atmosphere at room temperature. The measurement results of emission characteristics by applying DC voltage to the fabricated organic EL element are summarized in Table 3.

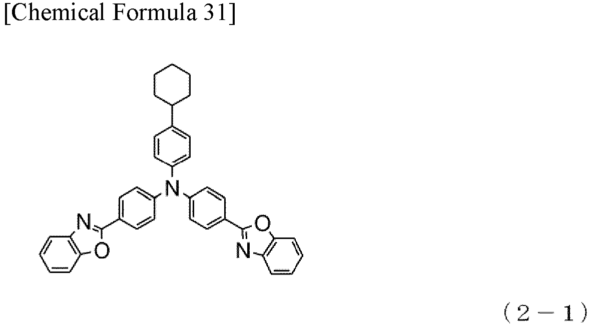

### [Comparative Example 2]

For comparison, an organic EL element was prepared under the same conditions as in Example 7, except that a comparative compound (2-2) represented by the following structural formula was used instead of the compound (1-18) of Example 1 to form the capping layer 10 having a film thickness of 60 nm. The characteristics of the fabricated organic EL element were measured in the atmosphere at room temperature. The measurement results of emission characteristics by applying DC voltage to the fabricated organic EL element are summarized in Table 3.

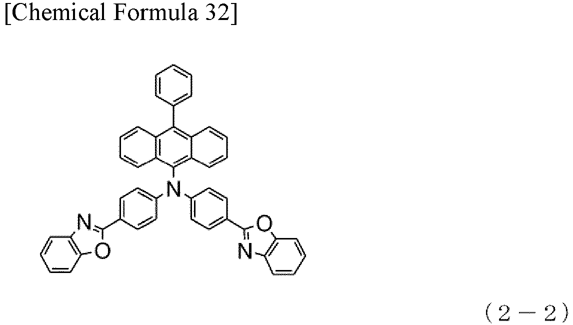

### [Comparative Example 3]

For comparison, an organic EL element was prepared under the same conditions as in Example 7, except that a comparative compound (2-3) of the following structural formula was used instead of the compound (1-18) of Example 1 to form the capping layer 10 having a film thickness of 60 nm. The characteristics of the fabricated organic EL element were measured in the atmosphere at room temperature. The measurement results of emission characteristics by applying DC voltage to the fabricated organic EL element are summarized in Table 3.

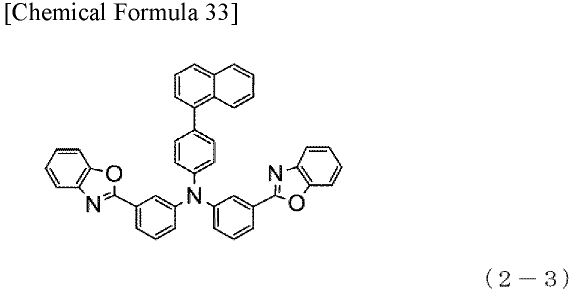

### [Comparative Example 4]

For comparison, an organic EL element was prepared under the same conditions as in Example 7, except that a comparative compound (2-4) represented by the following structural formula was used instead of the compound (1-18) of Example 1 to form the capping layer 10 having a film thickness of 60 nm. The characteristics of the fabricated organic EL element were measured in the atmosphere at room temperature. The measurement results of emission characteristics by applying DC voltage to the fabricated organic EL element are summarized in Table 3.

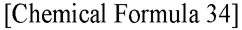

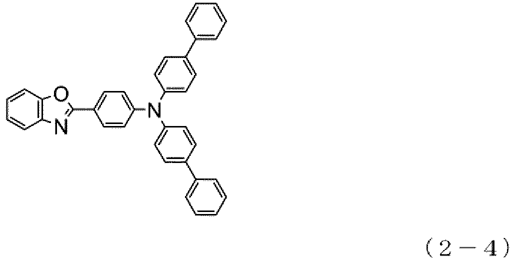

Element lifetimes of organic EL elements prepared in Examples 12 to 19 and Comparative Examples 1 to 4 were measured, and the results are summarized in Table 3. The element lifetime was measured as the time until the luminance decayed to 95% of an initial luminance which was set to 100%, when the element was driven at a constant current of 10 mA/cm².

**[Table 3]**

| | Capping layer | Voltage [V]₂ (@10 mA/cm²) | Luminance [cd/m²] (@10 mA/cm²) | Luminous efficiency [cd/A] (@10 mA/cm²) | Power efficiency [lm/W] (@10 mA/cm²) | Element lifetime 95% decay |
|---|---|---|---|---|---|---|
| Example 12 | Exemplified Compound (1-18) | 4.06 | 16671 | 166.89 | 129.33 | 365 hrs |
| Example 13 | Exemplified Compound (1-6) | 4.01 | 17154 | 171.58 | 134.48 | 386 hrs |
| Example 14 | Exemplified Compound (1-17) | 4.04 | 16321 | 163.22 | 126.95 | 377 hrs |
| Example 15 | Exemplified Compound (1-5) | 4.04 | 16559 | 165.75 | 128.95 | 368 hrs |
| Example 16 | Exemplified Compound (1-19) | 4.03 | 16510 | 165.21 | 128.97 | 389 hrs |
| Example 17 | Exemplified Compound (1-7) | 4.02 | 16770 | 167.79 | 131.18 | 369 hrs |
| Example 18 | Exemplified Compound (1-13) | 4.07 | 16419 | 164.37 | 126.91 | 382 hrs |
| Example 19 | Exemplified Compound (1-1) | 4.03 | 16997 | 170.09 | 132.59 | 384 hrs |
| Comparative Example 1 | Comparative Compound (2-1) | 4.02 | 13139 | 131.45 | 102.78 | 291 hrs |
| Comparative Example 2 | Comparative Compound (2-2) | 4.01 | 14625 | 146.30 | 114.55 | 302 hrs |
| Comparative Example 3 | Comparative Compound (2-3) | 4.06 | 13814 | 138.29 | 107.02 | 301 hrs |
| Comparative Example 4 | Comparative Compound (2-4) | 4.03 | 13022 | 130.31 | 101.68 | 257 hrs |

As shown in Table 3, the drive voltages at a current density of 10 mA/cm² are almost the same between the elements of Comparative Examples 1 to 4 using the comparative compounds (2-1), (2-2), (2-3), and (2-4) and the elements of Examples 12 to 19; however, luminance, luminous efficiency, power efficiency, and lifetime of the elements of Examples 12 to 19 were significantly improved compared with those of the elements of Comparative Examples 1 to 4 using the comparative compounds (2-1), (2-2), (2-3), and (2-4). This indicates that light extraction efficiency can be greatly improved by including, in the capping layer, a material having a high refractive index and suitably used for the organic EL element.

### INDUSTRIAL APPLICABILITY

As described above, the amine compound represented by general formula (1), which is suitably used as at least one of compounds (1-1), (1-6), and (1-18) in the organic EL element of the present invention, has a high absorption coefficient, has a high refractive index, can greatly improve the light extraction efficiency, and has a stable thin film state. Therefore, the amine compound is excellent as a compound for the organic EL element. By manufacturing an organic EL element using the compound, not only can high efficiency be obtained, but durability and light resistance can also be improved because the light of sunlight is absorbed and the material inside the element is not affected. Further, by using the compound having no absorption in each of the wavelength regions of blue, green, and red, the compound is particularly suitable for displaying a clear and bright image with good color purity. For example, the compound can be applied to home appliances and lighting.

### REFERENCE NUMBERS IN DRAWINGS:

1 Glass substrate
2. Metal anode
3. Hole injection layer
4. First hole transport layer
5. Second hole transport layer
6. Luminous layer
7. Electron transport layer
8. Electron injection layer
9. Cathode
10 Capping layer

## Claims

1. An organic electroluminescent element comprising at least an anode, a hole transport layer, a luminous layer, an electron transport layer, a cathode, and a capping layer in this order,
wherein a refractive index of a material of the capping layer is 1.90 or more at a wavelength of 500 nm to 570 nm;
wherein the capping layer comprises at least one compound selected from the group consisting of a compound (1-1) represented by the following formula (1-1), a compound (1-6) represented by the following formula (1-6), and a compound (1-18) represented by the following formula (1-18) 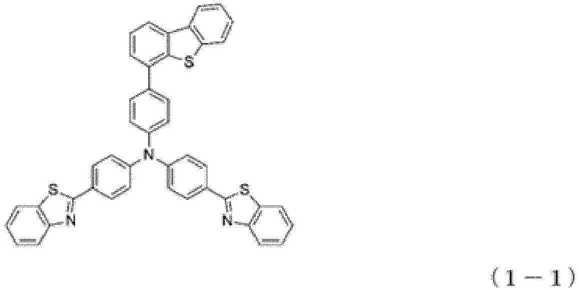 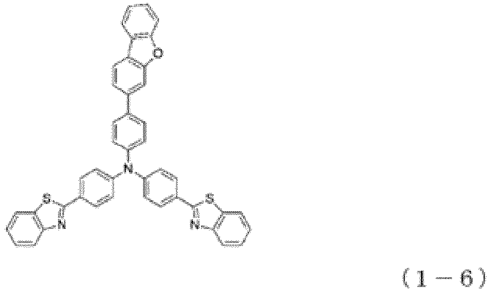 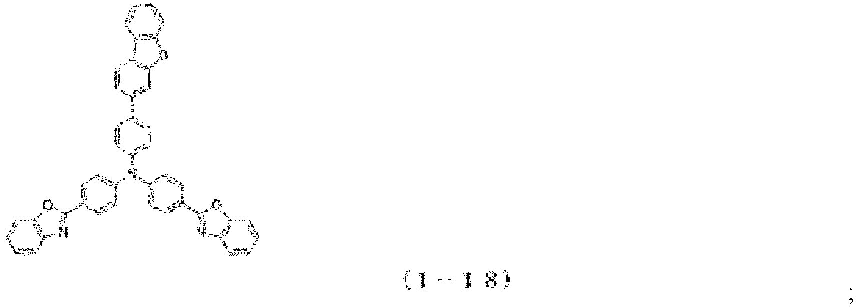 and
wherein the hole transport layer comprises an arylamine compound having two triphenylamine structures in a molecule and having a structure in which the two triphenylamine structures are linked by a single bond or a divalent group not containing a hetero atom.

2. The organic electroluminescent element according to claim 1, wherein a thickness of the capping layer is in a range of 30 nm to 120 nm.

## Patentansprüche

1. Organisches Elektrolumineszenzelement, umfassend mindestens eine Anode, eine Lochtransportschicht, eine Leuchtschicht, eine Elektronentransportschicht, eine Kathode und eine Deckschicht in dieser Reihenfolge,
wobei der Brechungsindex eines Materials der Deckschicht bei einer Wellenlänge von 500 nm bis 570 nm 1,90 oder mehr beträgt;
wobei die Deckschicht mindestens eine Verbindung, ausgewählt aus der Gruppe, bestehend aus einer Verbindung (1-1), dargestellt durch die folgende Formel (1-1), einer Verbindung (1-6), dargestellt durch die folgende Formel (1-6), und einer Verbindung (1-18), dargestellt durch die folgende Formel (1-18), umfasst 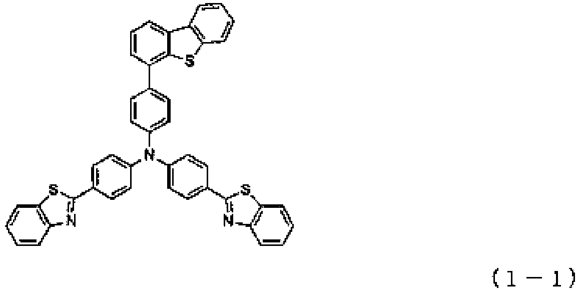 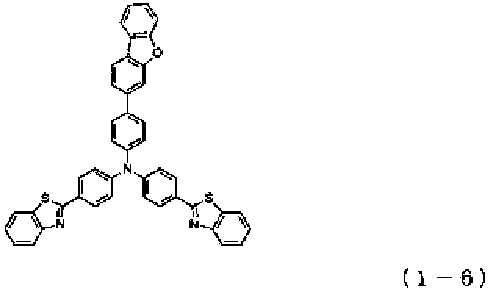 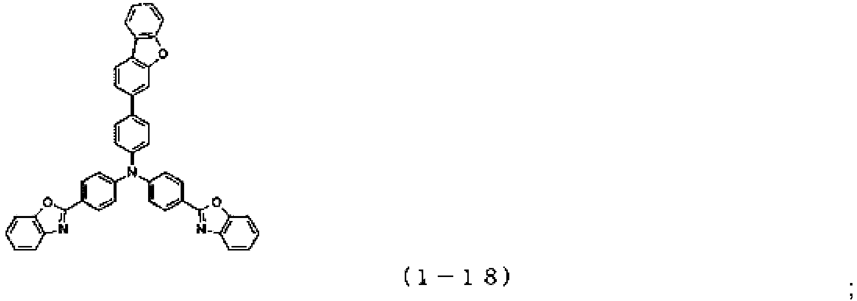 und
wobei die Lochtransportschicht eine Arylaminverbindung mit zwei Triphenylaminstrukturen in einem Molekül umfasst und eine Struktur aufweist, in der die zwei Triphenylaminstrukturen durch eine Einfachbindung oder eine zweiwertige Gruppe, die kein Heteroatom enthält, verbunden sind.

2. Organisches Elektrolumineszenzelement nach Anspruch 1, wobei eine Dicke der Deckschicht in einem Bereich von 30 nm bis 120 nm liegt.

## Revendications

1. Élément électroluminescent organique comprenant au moins une anode, une couche de transport de trous, une couche lumineuse, une couche de transport d'électrons, une cathode et une couche de recouvrement dans cet ordre,
dans lequel un indice de réfraction d'un matériau de la couche de recouvrement est de 1,90 ou plus à une longueur d'onde de 500 nm à 570 nm ;
dans lequel la couche de recouvrement comprend au moins un composé sélectionné parmi le groupe constitué d'un composé (1-1) représenté par la formule suivante (1-1), d'un composé (1-6) représenté par la formule suivante (1-6) et d'un composé (1-18) représenté par la formule suivante (1-18) 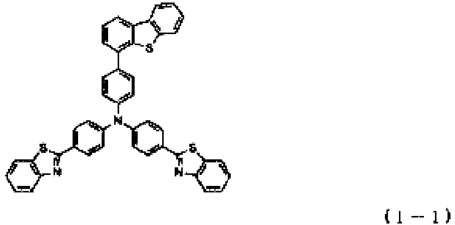 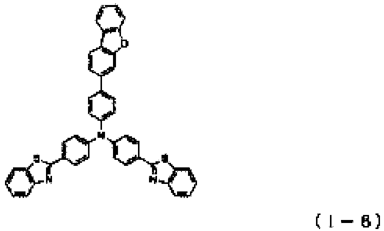 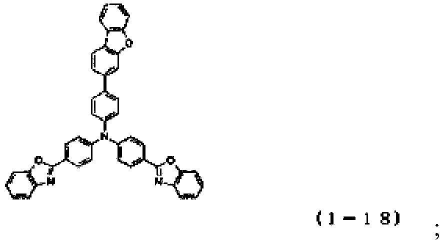 et
dans lequel la couche de transport de trous comprend un composé d'arylamine ayant deux structures de triphénylamine dans une molécule et ayant une structure dans laquelle les deux structures de triphénylamine sont liées par une liaison simple ou un groupe divalent ne contenant pas d'hétéroatome.

2. Élément électroluminescent organique selon la revendication 1, dans lequel une épaisseur de la couche de recouvrement est dans une plage de 30 nm à 120 nm.
